# EUROPEAN PATENT SPECIFICATION

(11) **EP 3 366 350 B1**
(45) Date of publication and mention of the grant of the patent: **09.07.2025**
(21) Application number: 17305199.6
(22) Date of filing: 23.02.2017
(51) Int. Cl.: A61N 7/02

(54) **A THERAPEUTIC TREATMENT DEVICE, A METHOD FOR DEFINING A SETUP AND A COMPUTER PROGRAM PRODUCT**
VORRICHTUNG ZUR THERAPEUTISCHEN BEHANDLUNG, VERFAHREN ZUR DEFINITION EINER EINSTELLUNG UND COMPUTERPROGRAMMPRODUKT
DISPOSITIF DE TRAITEMENT THÉRAPEUTIQUE, PROCÉDÉ PERMETTANT DE DÉFINIR UNE CONFIGURATION ET PRODUIT-PROGRAMME INFORMATIQUE

(43) Date of publication of application: 29.08.2018
(73) Proprietor: Theraclion SA, 92240 Malakoff (FR)
(72) Inventor: Grisey, Anthony, 78210 Saint Cyr l'Ecole (FR); Pechoux, Thierry, 75019 Paris (FR); Anquez, Jérémie, 75018 Paris (FR); Yon, Sylvain, 92220 Bagneux (FR)
(74) Representative: Hepp Wenger Ryffel AG

(56) References cited:
- WO-A2-03/042707
- JP-A- 2003 038 514
- JP-A- 2014 030 509

## Description

The present invention is directed to a therapeutic treatment device, a method for defining a setup, a computer program product as defined in the independent claims.

In particular, the invention concerns deviceswith high intensity focused ultrasounds (HIFU).

Conventionally, in the HIFU treatment, an acoustic treatment transducer emits concentrated acoustic waves onto a target tissue. These waves are absorbed by the tissue, which provokes a temperature rise in the tissue in the focal region. This temperature elevation in turn induces a local necrosis and thereby allows destruction of living tissue at a distance without any direct contact.

During the treatment, the HIFU beam is partially reflected by tissue interfaces such as the skin or by mechanical parts such as a balloon including contact gel or water. The reflection might result in energy being partially refocused and transmitted back onto the device.

In some conditions, which are deemed "harmful", the reflected energy can damage HIFU sensitive parts of the treatment head such as an imaging probe. WO 2011/157598 suggests providing a baffle mounted on the embedded imaging probe. However, such a baffle can reduce the quality of the images, interfere with the treatment HIFU beam or still provide an insufficient protection.

WO 03/042707 A2 relates to focused ultrasound systems, in particular to systems and methods to sense and locate reflective discontinuities and disturbances in ultrasound systems.

JP 2014 030509 A relates to ultrasound systems and reflection of ultrasound waves. The publication discloses systems comprising damage calculating units to calculate the damage caused by reflected ultrasound waves.

There is therefore a need for a device which is adapted to overcome at least some of the above mentioned disadvantages, in particular there is a need for a device in which damage of HIFU sensitive parts can be reliably prevented.

The problem is solved with a device according to the independent claim. In particular, the problem is solved with a device for therapeutic treatment of a target, in particular an organ or tissue. The device is operable in at least a detection mode during a detection phase and a treatment mode during a treatment phase. The device comprises a control for operating said device, a HIFU transducer for generating and transmitting ultrasound pulses to said target and at least one component which is sensitive to HIFU. In the detection mode, a processor is adapted to generate a harmful condition signal indicating whether or not there is a harmful condition in which reflected HIFU waves would impinge on said sensitive component during the treatment phase in such a manner as to transmit an amount of energy/power being harmful for the sensitive component. The control is adapted to define operation characteristics of said device for the treatment phase based on the harmful condition signal generated by said processor during the detection phase such that the reflected HIFU waves do not impinge on said component in such a manner as to transmit an amount of energy/power being harmful for the sensitive component in the treatment phase.

Preferably, the signal generated by the processor provides details about the energy/power impinging on the sensitive component. In particular, the signal might provide the following types of information:
1) Reflected power (total or impinging on the element) [in watts],
2) Degree of focusing of the reflected beam [area where the intensity of the reflected beam at the surface of the element is over a certain level, for example over 6dB of the maximum intensity],
3) Intensity of the reflected beam at the level of the component (maximum intensity or intensity map),
4) Signal representing the probability of damage to the element.

The adjustment of the operation characteristics can then be based on the information provided by the signal. The detailed signal allows for a better planning in adjustment avoiding multiple trial and error steps.

Alternatively, the signal generated by the processor might be such that it simply indicates if a harmful condition is present or not (yes/no signal). The adaptation of the operation characteristics may be made in a trial and error form in such a case.

The sensitive part might, for example, be an ultrasound imaging transducer. It might also be a portion of the treatment transducer itself, which can be a single or a multi-element transducer. There might also be other HIFU sensitive parts in a HIFU treatment head.

In the detection phase the sensitive part will not be damaged, which is explained in more detail hereinafter. The device according to the invention is adapted to detect harmful conditions in the detection phase and to set the operation characteristics accordingly to avoid damage of the sensitive component in a treatment phase, when HIFU waves are emitted by the treatment transducer.

Therewith, the device provides a reliable way to avoid damage of sensitive parts. There are different options to adapt the operations characteristics, which are explained in more detail hereinafter.

The HIFU transducer is, in one alternative, adapted to generate and emit detection ultrasound pulses with at least a first energy during the detection phase and treatment ultrasound pulses with at least a second energy higher than the first energy during the treatment phase. The device further comprises a sensor spatially related to the sensitive component. The sensor is adapted to detect said reflected detection ultrasound waves during said detection phase and adapted to provide data to said processor to allow recognition of said harmful condition.

In this embodiment, ultrasound waves are emitted with an energy, which will not result in a damage of the sensitive component in the detection phase. Therewith, the harmful conditions can reliably be recognized without a risk of damaging the sensitive component.

Further, preferably no tissue in the target region is modified by the acoustic energy delivered during the detection phase. The pulses in the detection phase are not intended to treat the tissue but to allow detection of harmful configurations.

If the detected reflected signal is above a certain threshold, the condition is considered as harmful. The threshold may depend on the specific characteristics of the sensitive component. The actual risk of a damage of the sensitive component depends on at least two factors: the energy of the reflected ultrasound beam and whether or not the beam is focalized on the sensitive component. The threshold is set so low that it can be guaranteed that there will be no damaging of the sensitive component during the treatment phase.

For imaging probes (imaging transducers), it is estimated that about 1%-10% of the energy of a treatment pulse is sufficient to damage the imaging probe (if focalized). Hence, if the sensitive component is an imaging probe and in the detection phase it is detected that at least 1% of the reflected waves impinge on the imaging probe in a focalized way, the condition is considered harmful.

If a condition is considered harmful, the operation characteristics are adapted. Therewith, it can be guaranteed that in the treatment phase, when ultrasound waves with higher energy are emitted, the reflected ultrasound waves will not damage the sensitive component.

Preferably, the first energy emitted during the probing phase is less than 10% of said second energy emitted during the treatment phase. The energy of pulses emitted in the probing phase might, for example, be 1% of the energy of pulses emitted in the treatment phase. Therewith, the sensitive part will not be harmed during the probing phase.

The sensor might be part of the sensitive component itself and adapted to detect an incoming acoustic signal. For example, the imaging transducer is adapted to detect ultrasonic waves in a certain bandwidth. In another example, one or several thermocouples provide information on the temperature elevation of the sensitive component.

If the sensitive component is the imaging transducer, the incoming acoustic pressure may directly be sensed by elements of the imaging transducer. Most ultrasound imaging transducers are particularly fragile. For example, if heated by the reflected ultrasound beam, a silicon lens may separate from the active elements thereby downgrading the image quality of the images provided by the imaging transducer.

Elements of the imaging transducers are usually connected to electronics in an ultrasound scanner which switch the elements to either emission mode (where imaging pulses are emitted by the elements) or a listening mode (where the elements are connected to amplifiers). In the listening mode, the signal detected by an element is known as the "RF signal". The harmful condition may be detected by the processor by analysing the RF signals and finding the spurious signals, i.e. only those signals which are related to the HIFU emission. If the RF signal is above a certain level, a harmful condition may be present.

HIFU waves tend to be reflected onto the central elements of the imaging transducer in a treatment device according to the invention. Therefore, only the central elements of the imaging transducer may have to be monitored to provide a reliable indication of harmful configurations. Furthermore, the identification of the elements where the spurious signal is the strongest gives an indication of the spatial distribution of the reflected waves, i.e. where the reflected wave is impinging on the imaging transducer.

Alternatively, the sensor is a separate sensor spatially related to the sensitive part. For example, an imaging transducer tuned at a frequency other than the HIFU frequency may not detect a potentially harmful acoustic beam. Hence, an external sensor could be provided to able to detect in the relevant frequency.

In order to provide reliable information of the condition, the sensor has to be spatially related to the sensitive component. Only therewith could the sensor and the processor estimate the reflected energy/power which will impinge on the sensitive component during the treatment phase.

The sensor is preferably at least one of the following, a therapy transducer in receive mode, a cavitation detector, a thermocouple or a pressure sensor. It is also possible to provide more than one sensor. If more than one sensor is used, the sensors are preferably different sensors selected from the above list of sensors.

The acoustic frequency of the detection pulse might be adapted to maximize the detection signal. For example, the HIFU transducer may be made up of piezoceramics which can be driven at their third harmonics with a reasonable efficiency coefficient. This is, for example, useful when the sensitive component is an ultrasound imaging probe because they are generally working at higher frequencies than therapy transducers. For the detection pulse the HIFU transducer will then be driven at its 3^{rd} harmonics to better match the spectral sensitivity of the imaging probe while for the treatment pulse the HIFU transducer will be driven at its 1^{st} harmonics.

Preferably, during the detection pulse, all sensing elements of the sensor (e.g. the imaging probe) are in "receive mode" so that reflection on the whole array is simultaneously monitored. Therewith, independent on the duration of the emitted pulse in the detection phase, all sensing elements of the sensor are able to detect reflected waves.

Alternatively, if it is not possible to control the sensor such that all its elements are in receiving mode (i.e. listening) in parallel, the detection pulse can be lengthened so that all the sensing elements of the sensor are in receiving mode at least once during the pulse. For example, with a B-mode ultrasonographer, the transducers (sensing elements) are in a receiving mode sequentially. In such a case, with a scanning frequency of 20 Hz, for example, the detection pulse duration could be extended to at least 50 ms, i.e. to cover at least one frame. Therewith, it is avoided that there is a harmful configuration present, which is not detected because one of the sensing element was not in listening mode during the pulse.

The maximum duration of a detection pulse is determined by the necessity of limiting its energy to avoid any damage. The detection pulse duration might therefore be extended to 100 or even up to 200 ms to cover at least one frame depending on the scanning frequency.

When the sensor can be used in active or passive mode, it is preferably switched to passive mode during the detection pulses. In the passive mode, the sensor is only sensing but not emitting any waves itself. Therewith, the fields of the HIFU transducer and the sensor do not interfere. Similarly all other acoustic sources are preferably turned off to avoid any interference of acoustic fields.

If the sensor cannot be switched to passive mode, the detection pulse should be strong enough to allow detection of the reflected signal in presence of other acoustic fields. Alternatively, if the other acoustic fields are not at the same frequency as the HIFU transducer, the signals can be separated by using spectral analysis.

It is also possible to detect a harmful condition using the image provided by the ultrasound scanner. The image is generated based on signals provided by the imaging probe. This mode is particularly useful when either the ultrasound scanner cannot be fully controlled by the HIFU device, or when the elements of the imaging transducer cannot be switched to receiving mode only or when it is not possible to synchronize the imaging scanning with the HIFU emission. Because in this cases, the RF signal as described hereinbefore is not available or at least not available for all elements.

It was observed that the potentially harmful reflection is clearly distinguishable from the noise generated by the HIFU emission. The potentially harmful reflection is displayed as a sharp cone at the relevant position. An automatic recognition of a harmful condition might therefore be carried out using image recognition methods. For example, a template of a triangle may be introduced. If the reflection is detected in the triangle, a harmful configuration is present.

The harmful condition signal is generated by the processor based on the ultrasonography images recorded during the detection pulse.

Furthermore, if the sensitive part is an imaging probe and a real-time image is displayed to a user, the image is preferably not displayed during the detection pulse(s) so that no artefact related to the emission of the detection pulse is visible in the image.

Preferably, if the position of the main reflecting portion can be estimated, the monitoring of the reflected signal is gated to take into account the travel time of the detection pulse from its emission until reaching the target. The time gate, i.e. the time where the signal is taken into account is the time between t_{back&forth} - εₑₐᵣₗᵢₑᵣ and t_{back&forth} + t_{detection} + εₗₐₜₑᵣ, wherein:
- t_{back&forth}:: estimated time for the wave to travel from the therapy transducer to the exposed part after a reflection on the main reflector portion;
- t_{detection}:: the duration of a detection pulse;
- εₑₐᵣₗᵢₑᵣ:: tunable margin (supplemental time accounting for the case where the reflector is closer than expected and)
- εₗₐₜₑᵣ:: tunable margin (supplemental time accounting for the case where the reflector is further away than expected).

A duration of the detection ultrasound pulses is preferably shorter than 1000 cycles, where a cycle is the inverse of the acoustic frequency of the detection pulse. Using short pulses enables the use of higher power. Therewith, the signal to noise ratio is improved, especially when the monitored signal is the acoustic pressure.

When the harmful condition is detected using the image provided by the ultrasound scanner, the duration of the detection ultrasound pulses is preferably shorter than 200 ms, preferably the duration is shorter than 100 ms. Alternatively, the duration might be expressed in cycles. The duration of the detection pulse is, when the harmful condition is detected using the image provided by the ultrasound scanner, preferably about 300'000 cycles if a frequency of 3 MHZ is used resulting (resulting in a duration of 100 ms).

Single short pulses enable the testing of one precise configuration. Such single short pulses are preferably used when the target and reflecting portions do not move. With non-moving portions it is sufficient to provide a single pulse, because the reflection will remain the same for each subsequent pulse.

If the target moves, for example due to breathing, several detection pulses are preferably sent prior to a treatment (ablation) pulse because the reflection might be different for subsequent pulse(s). Therewith, more, preferably all, relevant anatomical configurations can be evaluated. The duration of the detection pulses is preferably small compared to a characteristic time of the target motion. Conversely, the total duration of the detection pulses should be large compared to the characteristic time of the target motion to enable evaluation of the possible anatomical configurations. For example, the pulse sequence duration t_{sequence} could be set to: t_{sequence} = k x T_{breathing}, wherein k ≥ 1. T_{breathing} is the period between two subsequent breaths of air of the subject comprising the target.

This implies that the transducer has to be in the "firing"-position (i.e. in the position where the subsequence treatment pulse must be delivered) at least for the time t_{sequence} before the beginning of the treatment pulse. Therewith, it is ensured that the different configurations, which are present due to the breathing, can be estimated.

In case of a moving target, the criterion/criteria for changing a subsequent treatment pulse parameter can be adapted if a position is considered harmful. A position may be considered as harmful in a non-limiting way if one or more of the following is detected:
- any position tested during the sequence is harmful;
- at least X% of the positions tested in the pulse section are harmful.

In a second alternative, the processor is adapted to recognize said harmful condition based on a simulation of an acoustic field reflected by a boundary present at or near the target.

Instead of directly detecting reflected ultrasound waves with a sensor, the harmful condition is recognized through a simulation model. Therewith, it is not necessary to emit probing pulses on the patient. The simulation can be made on a computer, therewith reducing the time the patient has to be present for treatment.

In order to provide a reasonable simulation, the 3D geometry of the anatomical scene, or at least, of the main reflective interface should be known.

Preferably, the 3D geometry of the anatomical scene is acquired using a 3D imaging device such as ultrasound scanner, an X-Ray scanner or a magnetic resonance scanner or another method. Alternatively, the 3D geometry may be reconstructed based on a collection of 2D images of the anatomical scene. The acquiring can be performed at any time before the treatment or preferably before each therapy pulse.

Preferably the skin surface is detected to provide a 3D geometry. The skin surface might be described as a second degree surface (i.e. flat, spherical or ellipsoidal) by using best fitting algorithms, yielding a set of first order parameters (such as distance of the skin surface to the focus, tilt of the skin surface with respect to a main acoustic axis, radii of curvature of the skin surface). In addition a set of second order parameters may render how the actual surface departs from the second degree surface.

The first order parameters are used to predict the location of the reflected focus, using methods such as ray tracing or optical lens combination.

The second order parameters are used to predict the concentration of the reflected focus. For example, if the skin surface is very irregular, the reflected focus will not be sharp (i.e. reflected energy will be spread over a rather wide area). If the reflected focus is little or poorly concentrated, a higher ablation concentration is considered safe, i.e. not harmful.

If the 3D geometry moves (e.g. due to breathing), a collection of representative 3D sense is preferably computed.

In the different embodiments of the present invention, the control is adapted to adjust at least one of the following characteristics in said treatment phase as compared to the detection phase:
- Position of said HIFU transducer, preferably along a main propagation axis;
- Tilt or rotation of said HIFU transducer;
- Position of a shield element, preferably a baffle;
- Geometry of a mechanical reflector;
- Operation parameters of a phased array of the HIFU transducer;
- Stop or delay a HIFU pulse.

If a potentially harmful condition is detected, the HIFU transducer might be moved until it reaches a safe position. The HIFU transducer might be moved along the main propagation axis or along another axis.

Alternatively or additionally, the HIFU transducer might be tilted until it reaches a safe position.

Alternatively or additionally, the sensitive part can be protected with a mobile acoustic shielding subsystem, preferably a baffle. The mobile acoustic shielding subsystem is preferably only used when a harmful condition is detected. Alternatively, the subsystem can be moved independently from the therapy transducer to be placed in a safe condition.

Alternatively or additionally, a geometry of a mechanical reflector is changed when a potentially harmful condition is detected. For example, the geometry of an inflatable balloon ensuring acoustic coupling can be changed through inflating or deflating. Therewith, the skin geometry of the patient might also be modified if the pressure applied to the skin changes.

Alternatively or additionally, if the therapy transducer comprises a phased array, the amplitude and/or the phase of the transducer elements contributing to the therapy beam can be tuned. For example, the focus of the transducer can be electronically moved. Phase arrays provide secondary foci. Adaption of characteristics of the treatment phase might take into account the position and intensity of these secondary foci to ensure that the sensitive component is not damaged.

Alternatively or additionally, the HIFU pulses are stopped or delayed, if a potentially harmful condition is detected.

By changing at least one of the above mentioned characteristics, it can be ensured that the sensitive component is not damaged during the treatment phase.

The change of characteristics of one or several of the above mentioned options can rely on an optimization algorithm. For example, a gradient method where the derivative of the harmful condition signal vs each elementary motion is computed could provide the preferred direction of tilting or movement of the HIFU transducer to effectively achieve a safe position with a minimized change of characteristics. Therewith, a safe position can be achieved more easily without the need for "trial and error". Details of optimization algorithms are also described in Kelley, C. T. (1999); "Iterative methods for optimization"; Society for Industrial and Applied Mathematics.

If the focus location can be detected during a detection pulse, the pulse parameter adaption could be done according to the following steps:
- Tilting the therapy transducer by a small angle around several axes,
- Recoding the displacement of the focus for each direction,
- Selecting the axis for which the focus moves closer to the border of the sensitive component,
- Tilting the transducer along this axis by an amount which theoretically moves the focus outside the sensitive component,
- Performing another detection pulse,
- Repeating the process if the focus is still inside the sensitive component,
- Switching to treatment mode if the focus is outside the sensitive component.

Therewith, it can be ensured that the sensitive component is not damaged.

Modification of the pulse parameters may require a validation by the user as they modify the treatment planning. Alternatively, some authorized modifications can be considered minor and to not require a validation.

Following a "pulse adaption", a new sequence of probing pulses may be emitted to ensure that the changes did not result in a harmful configuration.

The operations might be carried out automatically with a suitably programmed computer.

Preferably, the device according to the present invention further comprises synchronization means to synchronize said transmittal of ultrasound waves with an anatomical movement such as breathing.

Therewith, it can be assured that the sensitive part is not damaged. The pulse emission during the treatment phase might be synchronized such as to be switched off when the anatomy is in a position which was considered as harmful.

Alternatively or additionally, the transducer is moved in a predefined way when the anatomy is in a position which was estimated harmful.

Alternatively or additionally, the amplitude and/or phase of the transducer elements are temporarily modified in a predefined way when the anatomy is in a position which was estimated harmful.

The present invention is also directed to a method for defining a setup for the operation of a therapeutic treatment device with a HIFU transducer adapted to generate and transmit ultrasound waves to a target. A therapeutic treatment device as described hereinbefore is used. The method comprises the steps of:
- Recognizing during a detection mode with a processor if a harmful condition is present in which reflected ultrasound waves would impinge on a HIFU sensitive component in such a manner as to transmit an amount of energy/power being harmful for the sensitive part,
- Setting operation characteristics of said device for a treatment phase such that reflected treatment ultrasound waves do not impinge on said component in such a manner as to transmit an amount of energy/power being harmful for the sensitive part during the treatment phase.

Preferably, the method further comprises the steps of:
- Emitting detection ultrasound waves with a first energy with said HIFU transducer during said detection mode,
- Detecting reflected ultrasound waves with a sensor spatially related to the sensitive component, to allow said processor to recognize said harmful condition,
- Emitting treatment ultrasound waves with a second energy with said HIFU transducer during a treatment phase after said operation characteristics have been set.

Alternatively, the method further comprises the steps of:
- Acquiring a 3D geometry of an anatomical scene of the target,
- Simulating the reflected acoustic field for multiple, preferably every possible, pulse positions and multiple, preferably every possible, 3D configurations of the anatomical scene,
- Calculating the intensity and the energy of the reflected acoustic pulse with said processor in order to recognize said harmful condition(s).

Preferably, in a method according to the present invention, said detection mode is performed before each treatment pulse is emitted in said treatment phase.

A method for treating tissue with HIFU from a therapeutic treatment device, with a HIFU transducer adapted to generate and transmit ultrasound waves to a target, is described. Preferably a therapeutic treatment device as described hereinabove is used. The method comprises the steps of:
- Recognizing during a detection mode with a processor if a harmful condition is present in which reflected ultrasound waves would impinge on a HIFU sensitive component in such a manner as to transmit an amount of energy/power being harmful for the sensitive part,
- Setting operation characteristics of said device for a treatment phase such that reflected treatment ultrasound waves do not impinge on said component in such a manner as to transmit an amount of energy/power being harmful for the sensitive part during the treatment phase,
- Emitting treatment HIFU waves during a treatment phase.

Preferably, the method further comprises the steps of:
- Emitting detection ultrasound waves with a first energy with said HIFU transducer during said detection mode,
- Detecting reflected ultrasound waves by a sensor to allow said processor to recognize said harmful condition,
Said treatment HIFU waves have a second energy higher than the first energy.

Alternatively, the method further comprises the steps of:
- Acquiring a 3D geometry of an anatomical scene of the target,
- Simulating the reflected acoustic field for multiple, preferably every possible, positions of the treatment head and multiple, preferably every possible, 3D configurations of the anatomical scene,
- Calculating the energy and the spatial distribution of energy of the reflected acoustic pulse potentially impinging on the sensitive component, with said processor in order to recognize said harmful condition(s)

The invention is also directed to a computer program product comprising software code portions for performing the steps of a method as described hereinabove, when the product is run on a computer.

Further aspects of the invention are described with reference to the following figures. The figures schematically disclose:
- Fig. 1a:: A device according to the invention in a probing phase;
- Figs 1b to 1d:: The device according to Figure 1a in the treatment phase;
- Fig. 2:: A pulse sequence with synchronization of detection and imaging pulses;
- Fig. 3:: A flowchart disclosing a method according to the invention;
- Fig. 4: A device according to the present invention;
- Fig. 5: Ultrasound images indicating a non-harmful and a harmful configuration.

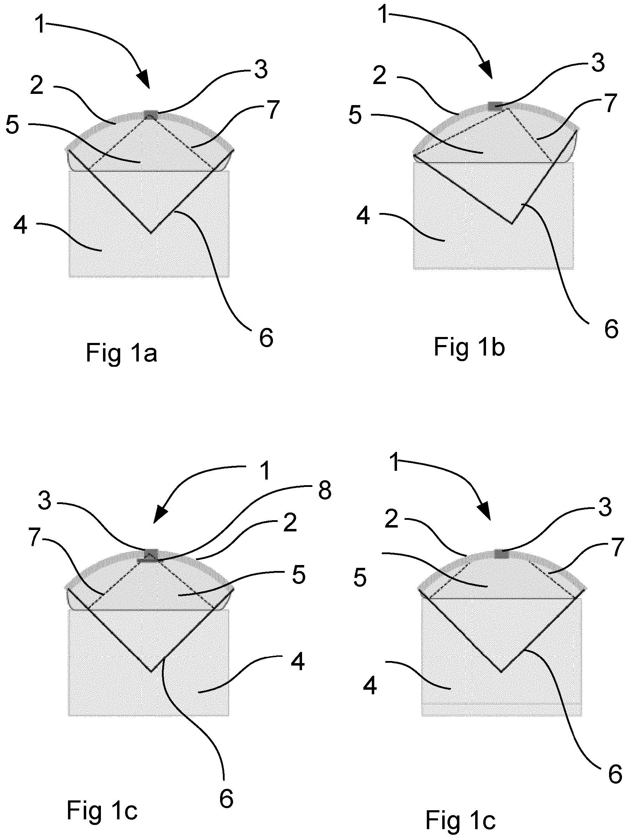

Figures 1a to 1d each disclose a therapeutic treatment device 1 according to the invention in a probing phase (1a) and a treatment phase (1b to 1d). The device comprises a HIFU treatment transducer 2 and an imaging probe 3. The imaging probe 3 is sensitive to HIFU and could therefore be damaged by HIFU reflected on a surface.

The treatment transducer 2 is adapted to emit a HIFU beam 6 towards a patient 4. The HIFU beam 6 has a focal point in a target of the patient 4. Between the HIFU transducer 2 and the patient 4 a coupling membrane 5 filled with water is arranged. A portion of the HIFU beam 6 is reflected at boundary of the patient 4. The reflected beam 7 could damage the imaging probe 3 if impinging on it.

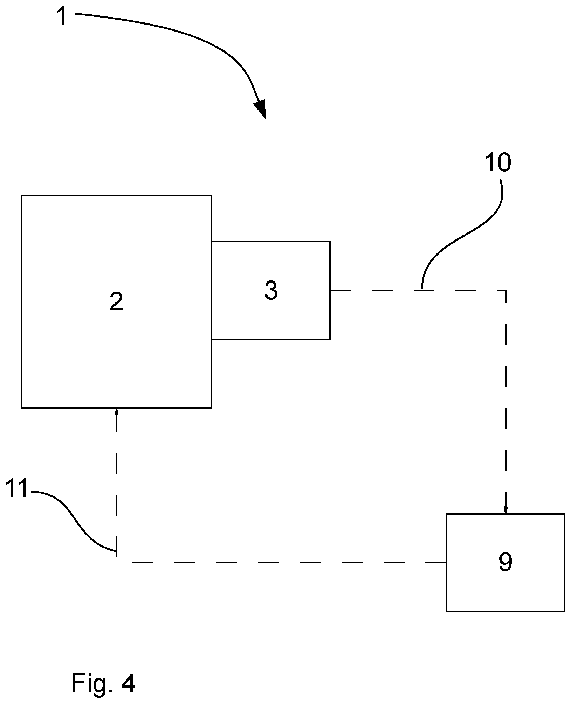

In 1a, the device 1 is shown in the probing phase. The treatment transducer 2 emits an ultrasound beam 6 with pulses with a first energy being only 1% of the energy of a treatment pulse. This energy is too low to damage the imaging probe 3 and to change tissue characteristics in the target of the patient 4. The imaging probe 3 (including its electronics usually included in an ultrasound scanner) is adapted to sense if the reflected ultrasound beam 7 is impinging on the imaging probe 3. The data of the imaging probe 3 is provided to a processor 9 (see Figure 4), which calculates if this impinging potentially harmful, i.e. if a harmful condition will be present during a subsequent treatment pulse. In the probing phase shown in Figure 1a, the reflected ultrasound beam 7 meets in a focal point on the imaging probe 3 and a potentially harmful amount of energy could be reflected on the imaging probe 3 in the treatment phase. Hence, a harmful configuration is present. The data 10 (see Figure 4) of the imaging probe 3 are provided to a processor 9 which generates a harmful condition signal 11 (see Figure 4), indicating if a harmful condition is present. Based on this signal, the operation characteristics for the treatment phase are adapted.

In the device 1 of figure 1b the adaption of the operation characteristics is a tilting of the treatment transducer 2 along a spherical axis such that the focal point of the ultrasound beam 6 in the patient 4 is not changed but the focal point of the reflected beam 7 is changed to not lie anymore in the imaging probe 3. Therewith, the reflected beam 7 cannot damage the imaging probe 3.

In the device 1 of figure 1c, the adaption of the operation characteristics is movement of a mobile baffle 8 in a shielding position. The mobile baffle 8 prevents the reflected beam 7 from impinging on the imaging probe 3. Therewith, the reflected beam 7 cannot damage the imaging probe 3.

In the device 1 of figure 1d, the adaption of the operation characteristics is a motion of the treatment transducer 2 along the longitudinal axis of the emitted ultrasound beam towards the target, such that the reflected beam 7 does not concentrate anymore onto the imaging probe 3. Therewith, the reflected beam 7 cannot damage the imaging probe 3.

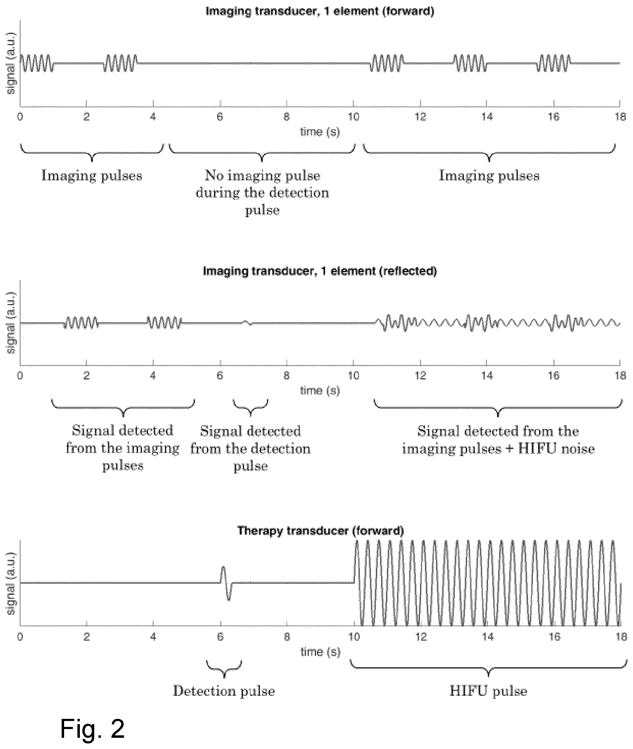

Figure 2 shows a pulse sequence with the synchronization of detection and imaging pulses. This allows detecting HIFU pulses without background noise. In the first (uppermost) illustration of figure 2, the emission of imaging pulses is disclosed. No imaging pulses are emitted during the emission of detection pulses by the treatment transducer 2. In the second (middle) illustration of figure 2, the sensing by the imaging transducer 2 is disclosed. The sensing of the detection pulse is not interfered by the imaging pulse (signal detected from the detection pulse). During treatment, imaging pulses and HIFU pulses are detected (Signal detected from the imaging pulses + HIFU pulses). In the third (bottommost) illustration of Figure 2, the emission of ultrasound waves of the HIFU transducer 2 is disclosed. A detection pulse is emitted during the detection phase and detected by the imaging probe 3. HIFU pulses with a higher energy are emitted during the treatment phase.

The synchronization allows the detection of reflected detection pulses without noise generated by imaging pulses.

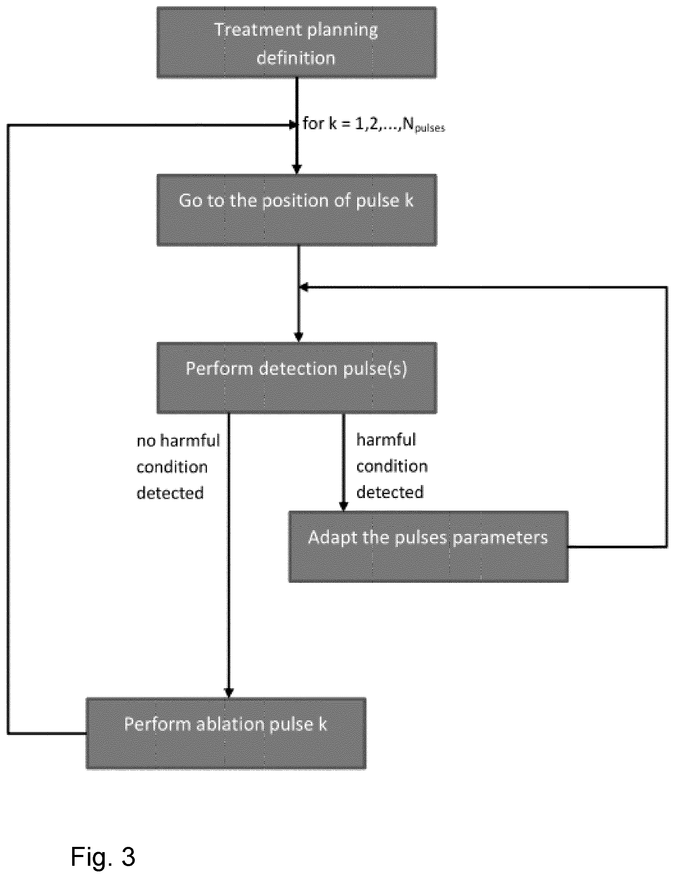

Figure 3 discloses a flowchart of a method according to the invention. First the treatment is planned with k treatment pulses. The treatment transducer 3 is then moved to the position of pulse k and at least one detection pulse is emitted. If a harmful configuration is detected by the sensor and the processor 9, operation characteristics are changed and at least one further detection pulse is emitted. This is repeated until no harmful configuration is detected by the sensor and the processor 9. If no harmful configuration is detected, ablation (treatment) pulses are emitted by the treatment transducer 2.

Figure 4 discloses a device 1 according to the present invention. The device 1 comprises a treatment transducer 2 and an imaging probe 3. The treatment transducer 3 is adapted to generate probing pulses 6 (see Figure 1). The reflected pulses 7 (see Figure 1) are sensed by an imaging probe 3. The imaging probe 3 provides the data along arrow 10 to a processor 9. The processor calculates with the data if the present configuration is harmful, i.e. in the present configuration reflected HIFU waves emitted by the treatment transducer 2 in the subsequent treatment phase would damage the imaging probe 3. The processor 9 then provides a signal along arrow 11 to the treatment transducer 2 indicating if a harmful configuration is present or not. If no harmful configuration is present, the treatment transducer 2 will start emitting HIFU treatment pulses. If a harmful configuration is present, the operations characteristics of the treatment transducer 2 are adapted new probing pulses 6are emitted by the treatment transducer 2. The cycle is repeated until no harmful configuration is detected anymore by the processor 9.

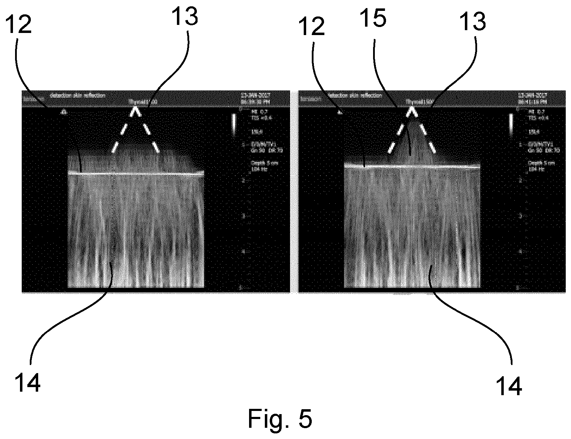

Figure 5 discloses ultrasound images indicating a non-harmful (left hand side) and a harmful (right hand side) configuration. The images can be used in a method to automatically detect a harmful configuration. The images of Figure 5 are generated with the following materials and settings:
∘ EchoPulse with Terason 3300; The imaging array was a 15L4V 128 elements mounted on treatment head VTU SN 309
∘ no baffle was mounted to protect the imaging array;
∘ water tank with 3cm thick silicone slab positioned over the bottom;
∘ HIFU settings for the emission of the detection pulses:
   ▪ Acoustic frequency: 3MHz;
   ▪duration of emission: 100ms at 3MHz (i.e. 300,000 cycles per pulse)
   ▪ HIFU power: 20W
   ▪→ Pulse energy 2J (for comparison, a treatment pulse is typically 100W x 4s, i.e. 400J, meaning that these detection pulses carry less than 1% of the energy of a treatment pulse)
∘ US Scanner The ultrasound system is a "usmart 3200T" from Terason; settings:
   ▪ Persistence: 0;
   ▪ compound mode and harmonic modes: off
   ▪ One focus only to optimize frame rate (104Hz)
   ▪ Depth of field of view: 5cm
∘ Positioning of Echopulse's treatment head in the tank:
   ▪ Focus located at variable distance, so that reflection of HIFU by surface of silicone slab roughly focalizes on imaging probe

For each of 15 positions of the treatment head of the HIFU transducer 2 (see Fig. 1, the silicone slab representing patient 4) relative to the surface of the silicone slab, a test pulse was emitted by the HIFU transducer 2. Ultrasound images provided by the scanner are recorded during the pulse, yielding in 15 sequences of images. The most significant image of each sequence (i.e. the one synchronous with the test pulse) is extracted from each sequence.

In Figure 5, two exemplary images are shown. The white line 12 is the result of the echo from the emission of the elements in the imaging array. The noise 14 (grey areas) results from the HIFU emission. The noise pattern in the proximal area (above the white line) is dependent on the position of the treatment head.

In the image on the right hand side, a harmful configuration is detected. The potentially harmful reflected ultrasound is clearly identified at 14-17 mm by a noise pattern in the shape of a sharp cone 15. The cone is the result of the beam formation in reception. On the left hand side, no cone is displayed indicating that no harmful configuration is present.

The image allows an automatic detection of a harmful configuration. Therefore, a template of a triangle 13 is matched to the grey levels in the proximal (upper) part of the image. On the right hand side the triangle 13 basically matches the cone 15. If a cone 15 and hence a harmful configuration is present, in the area in the triangle noise is displayed. This directly indicates a harmful configuration. If the triangle remains empty, no harmful configuration is present and treatment pulses can be emitted.

## Claims

1. A device (1) with a processor for therapeutic treatment of a target, in particular an organ or tissue, wherein the device (1)
is operable in at least a detection mode during a detection phase and a treatment mode during a treatment phase, said device comprising:
- a control for operating said device,
- a HIFU transducer (2) for generating and transmitting ultrasound pulses (6) to said target,
- at least one component (3) which is sensitive to HIFU, in particular an imaging transducer,
wherein in the detection mode, the processor (9) is adapted to generate a harmful condition signal indicating whether or not there is a harmful condition in which reflected HIFU waves (7) would impinge on said sensitive component (3) in such a manner as to transmit an amount of energy/power being harmful for the sensitive component (3),
wherein said control is adapted to define operation characteristics of said device (1) for the treatment phase based on the harmful condition signal generated by said processor (9) during the detection phase such that the reflected HIFU waves (7) do not impinge on said component (3) in such a manner as to transmit an amount of energy being harmful for the sensitive component (3) in the treatment phase,
wherein said control is adapted to adjust at least one of the following characteristics in said treatment phase as compared to the detection phase:
- Position of said HIFU transducer (2), preferably along a main propagation axis;
- Tilt of said HIFU transducer (2);
- Position of a shield element (8), preferably a baffle;
- Geometry of a mechanical reflector (5);
- Operation parameters of a phased array of the HIFU transducer (2);
- Stop or delay a HIFU pulse
**characterized in that** either
- the HIFU transducer (2) is adapted to generate and emit detection ultrasound pulses with at least a first energy during the detection phase and treatment ultrasound pulses with at least a second energy higher than the first energy during the treatment phase, the device further comprising a sensor spatially related to the sensitive component (3), wherein the sensor is adapted to detect said reflected detection ultrasound waves (7) during said detection phase and adapted to provide data to said processor (9) to allow recognition of said harmful condition, or
- said processor (9) is adapted to recognize said harmful condition based on a simulation of an acoustic field reflected by a boundary present at or near the target.

2. The device according to claim 1, further comprising synchronization means to synchronize said transmittal of ultrasound waves with an anatomical movement such as breathing.

3. The device according to claim 2, wherein said synchronization comprises at least one of the following operations:
- Stopping of ultrasound transmittal;
- Movement of the HIFU transducer (2);
- Modification of parameters of the HIFU transducer (2).

4. A method for defining a setup for the operation of a therapeutic treatment device (1) with a HIFU transducer (2) adapted to generate and transmit ultrasound waves (6) to a target, the therapeutic treatment device being a therapeutic treatment device according to one of the claims 1 to 3, comprising the steps of:
- Recognizing during a detection mode with a processor (9) if a harmful condition is present in which reflected ultrasound waves (7) would impinge on an HIFU sensitive component (3) in such a manner as to transmit an amount of energy/power being harmful for the sensitive component (3),
- Setting operation characteristics of said device for a treatment phase such that reflected treatment ultrasound waves (7) do not impinge on said sensitive component (3) in such a manner as to transmit an amount of energy/power being harmful for the sensitive component (3) during the treatment phase.

5. The method according to claim 4, further comprising the steps of:
- Acquiring a 3D geometry of an anatomical scene of the target,
- Simulating the reflected acoustic field for multiple, preferably every possible, pulse positions and multiple, preferably every possible, 3D configurations of the anatomical scene,
- Calculating the intensity and the energy of the reflected acoustic pulse with said processor (9) in order to recognize said harmful condition(s).

6. A computer program product comprising software code portions for performing the steps of a method according to one of the claims 4 or 5, when the product is run on a computer comprised in a device according to one of the claims 1 to 3.

## Patentansprüche

1. Vorrichtung (1) mit einem Prozessor zur therapeutischen Behandlung eines Targets, insbesondere eines Organs oder Gewebes, wobei die Vorrichtung (1)
in mindestens einem Detektionsmodus während einer Detektionsphase und einem Behandlungsmodus während einer Behandlungsphase betreibbar ist, wobei die Vorrichtung umfasst:
- eine Steuerung zur Bedienung der Vorrichtung,
- einen HIFU-Wandler (2) zur Erzeugung und Übertragung von Ultraschallimpulsen (6) auf das Target,
- mindestens ein für HIFU empfindliches Bauteil (3), insbesondere einen bildgebenden Wandler,
wobei in dem Detektionsmodus der Prozessor (9) angepasst ist, um ein schädliches Zustandssignal zu erzeugen, das anzeigt, ob es einen schädlichen Zustand gibt oder nicht, in dem zurückreflektierte HIFU-Wellen (7) auf die empfindliche Komponente (3) in einer solchen Weise auftreffen würden, dass eine Energie-/Leistungsmenge übertragen wird, die für die empfindliche Komponente (3) schädlich ist,
wobei die Steuerung geeignet ist, die Betriebscharakteristiken der Vorrichtung (1) für die Behandlungsphase auf der Grundlage des schädlichen Zustandssignals zu definieren, das von dem Prozessor (9) während der Detektionsphase erzeugt wird, so dass die zurückreflektierten HIFU-Wellen (7) nicht auf die Komponente (3) in einer solchen Weise auftreffen, dass sie eine Energiemenge übertragen, die für die empfindliche Komponente (3) in der Behandlungsphase schädlich ist, wobei die Steuerung geeignet ist, mindestens eine der folgenden Eigenschaften in der Behandlungsphase im Vergleich zur Detektionsphase einzustellen:
- Position des HIFU-Wandlers (2), vorzugsweise entlang einer Hauptausbreitungsachse;
- Neigung des HIFU-Wandlers (2);
- Position eines Abschirmelements (8), vorzugsweise eines Leitblechs;
- Geometrie eines mechanischen Reflektors (5);
- Betriebsparameter eines Phased Array des HIFU-Wandlers (2);
- Anhalten oder Verzögern eines HIFU-Pulses
**dadurch gekennzeichnet, dass** entweder
- der HIFU-Wandler (2) so beschaffen ist, dass er während der Detektionsphase Detektions-Ultraschallimpulse mit mindestens einer ersten Energie und während der Behandlungsphase Behandlungs-Ultraschallimpulse mit mindestens einer zweiten Energie, die höher als die erste Energie ist, erzeugt und aussendet, wobei die Vorrichtung ferner einen Sensor umfasst, der räumlich auf das empfindliche Bauteil (3) ausgerichtet ist, wobei der Sensor so beschaffen ist, dass er die reflektierten Detektions-Ultraschallwellen (7) während der Detektionsphase detektiert, und so beschaffen ist, dass er dem Prozessor (9) Daten bereitstellt, um die Erkennung des schädlichen Zustands zu erleichtern, oder
- der Prozessor (9) geeignet ist, den schädlichen Zustand auf der Grundlage einer Simulation eines akustischen Feldes zu erkennen, das von einer am oder in der Nähe des Ziels vorhandenen Grenze reflektiert wird.

2. Vorrichtung nach Anspruch 1, die ferner Synchronisationsmittel umfasst, um die Übertragung von Ultraschallwellen mit einer anatomischen Bewegung wie dem Atmen zu synchronisieren.

3. Vorrichtung nach Anspruch 2, wobei die Synchronisation mindestens einen der folgenden Vorgänge umfasst:
- Unterbrechung der Ultraschall-Übertragung;
- Bewegung des HIFU-Wandlers (2);
- Änderung der Parameter des HIFU-Wandlers (2).

4. Verfahren zum Definieren eines Aufbaus für den Betrieb einer therapeutischen Behandlungsvorrichtung (1) mit einem HIFU-Wandler (2), der geeignet ist, Ultraschallwellen (6) zu erzeugen und auf ein Target zu übertragen, wobei die therapeutische Behandlungsvorrichtung eine therapeutische Behandlungsvorrichtung nach einem der Ansprüche 1 bis 3 ist, umfassend die Schritte:
- Erkennen während eines Detektionsmodus mit einem Prozessor (9), ob ein schädlicher Zustand vorliegt, in dem reflektierte Ultraschallwellen (7) auf eine HIFU-empfindliche Komponente (3) in einer solchen Weise auftreffen würden, dass eine Energie-/Leistungsmenge übertragen wird, die für die empfindliche Komponente (3) schädlich ist,
- Einstellen der Betriebscharakteristiken der Vorrichtung für eine Behandlungsphase, so dass reflektierte Behandlungs-Ultraschallwellen (7) nicht auf das empfindliche Bauteil (3) in einer Weise auftreffen, dass eine Energie-/Leistungsmenge übertragen wird, die für das empfindliche Bauteil (3) während der Behandlungsphase schädlich ist.

5. Verfahren nach Anspruch 4, das ferner die folgenden Schritte umfasst:
- Erfassen einer 3D-Geometrie einer anatomischen Szene des Targets,
- Simulation des reflektierten akustischen Feldes für mehrere, vorzugsweise alle möglichen, Pulspositionen und mehrere, vorzugsweise alle möglichen, 3D-Konfigurationen der anatomischen Szene,
- Berechnung der Intensität und der Energie des zurückgeworfenen akustischen Impulses mit dem Prozessor (9), um die schädliche(n) Bedingung(en) zu erkennen.

6. Computerprogrammprodukt, das Software-Code-Teile zur Durchführung der Schritte eines Verfahrens nach einem der Ansprüche 4 oder 5 umfasst, wenn das Produkt auf einem Computer ausgeführt wird, der in einer Vorrichtung nach einem der Ansprüche 1 bis 3 enthalten ist.

## Revendications

1. Dispositif (1) avec processeur pour le traitement thérapeutique d'une cible, en particulier d'un organe ou d'un tissu, dans lequel le dispositif (1)
peut fonctionner au moins en mode de détection pendant une phase de détection et en mode de traitement pendant une phase de traitement, ledit dispositif comprenant :
- une commande pour faire fonctionner ledit dispositif,
- un transducteur HIFU (2) pour générer et transmettre des impulsions ultrasonores (6) à ladite target, au moins un composant (3) sensible aux HIFU, en particulier un transducteur d'imagerie,
dans lequel, dans le mode de détection, le processeur (9) est adapté pour générer un signal de condition nuisible indiquant s'il existe ou non une condition nuisible dans laquelle les ondes HIFU (7) réfléchies empiéteraient sur ledit composant sensible (3) de manière à transmettre une quantité d'énergie/puissance étant nuisible pour le composant sensible (3),
dans lequel ladite commande est adaptée pour définir les caractéristiques de fonctionnement dudit dispositif (1) pour la phase de traitement sur la base du signal de condition nocive généré par ledit processeur (9) au cours de la phase de détection de sorte que les ondes HIFU (7) réfléchies n'empiètent pas sur ledit composant (3) de manière à transmettre une quantité d'énergie nocive pour le composant sensible (3) au cours de la phase de traitement,
dans lequel ladite commande est adaptée pour ajuster au moins l'une des caractéristiques suivantes dans ladite phase de traitement par rapport à la phase de détection :
- Position dudit transducteur HIFU (2), de préférence le long d'un axe de propagation principal ;
- Inclinaison dudit transducteur HIFU (2);
- Position d'un élément de blindage (8), de préférence un déflecteur ;
- Géométrie d'un réflecteur mécanique (5);
- Paramètres de fonctionnement d'un réseau phasé du transducteur HIFU (2);
- Arrêter ou retarder une impulsion HIFU
**caractérisés par le fait que** soit
- le transducteur HIFU (2) est adapté pour générer et émettre des impulsions ultrasonores de détection avec au moins une première énergie pendant la phase de détection et des impulsions ultrasonores de traitement avec au moins une deuxième énergie plus élevée que la première énergie pendant la phase de traitement, le dispositif comprenant en outre un capteur spatialement relié au composant sensible (3), dans lequel le capteur est adapté pour détecter lesdites ondes ultrasonores de détection réfléchies (7) pendant ladite phase de détection et adapté pour fournir des données audit processeur (9) afin de permettre la reconnaissance de ladite condition nuisible, ou
- ledit processeur (9) est adapté pour reconnaître ladite condition nuisible sur la base d'une simulation d'un champ acoustique réfléchi par une limite présente au niveau ou à proximité de la cible.

2. Le dispositif selon la revendication 1, comprenant en outre des moyens de synchronisation pour synchroniser ladite transmission d'ondes ultrasonores avec un mouvement anatomique tel que la respiration.

3. Le dispositif selon la revendication 2, dans lequel ladite synchronisation comprend au moins l'une des opérations suivantes :
- Arrêt de la transmission des ultrasons ;
- Mouvement du transducteur HIFU (2) ;
- Modification des paramètres du transducteur HIFU (2).

4. Procédé de définition d'une configuration pour le fonctionnement d'un dispositif de traitement thérapeutique (1) avec un transducteur HIFU (2) adapté pour générer et transmettre des ondes ultrasonores (6) à une target, le dispositif de traitement thérapeutique étant un dispositif de traitement thérapeutique selon l'une des revendications 1 à 3, comprenant les étapes de :
- Reconnaître pendant un mode de détection avec un processeur (9) si une condition nuisible est présente dans laquelle les ondes ultrasonores réfléchies (7) impacteraient un composant sensible HIFU (3) de manière à transmettre une quantité d'énergie/puissance nuisible pour le composant sensible (3),
- Réglage des caractéristiques de fonctionnement dudit dispositif pour une phase de traitement de telle sorte que les ondes ultrasonores de traitement réfléchies (7) n'atteignent pas ledit composant sensible (3) de manière à transmettre une quantité d'énergie/puissance nocive pour le composant sensible (3) pendant la phase de traitement.

5. La méthode selon la revendication 4, comprenant en outre les étapes suivantes :
- Acquisition d'une géométrie 3D d'une scène anatomique de la target,
- Simulation du champ acoustique réfléchi pour plusieurs positions d'impulsion, de préférence toutes possibles, et plusieurs configurations 3D, de préférence toutes possibles, de la scène anatomique,
- Calculer l'intensité et l'énergie de l'impulsion acoustique réfléchie à l'aide dudit processeur (9) afin de reconnaître la (les) condition(s) nocive(s).

6. Produit programme d'ordinateur comprenant des por-tions de code logiciel pour l'exécution des étapes d'un procédé selon l'une des revendications 4 ou 5, lorsque le produit est exécuté sur un ordinateur compris dans un dispositif selon l'une des revendications 1 à 3.
